# EUROPEAN PATENT SPECIFICATION

(11) **EP 0 528 015 B1**
(45) Date of publication and mention of the grant of the patent: **07.10.1998**
(21) Application number: 92907693.3
(22) Date of filing: 21.02.1992
(51) Int. Cl.: A61C 8/00, A61C 7/00, A61B 17/16

(54) **BONE ANCHOR**
KNOCHENANKER
ANCRAGE OSSEUX

(30) Priority: 25.02.1991 US 659680; 19.11.1991 US 792855
(43) Date of publication of application: 24.02.1993
(73) Proprietor: Nobel Biocare AB, 402 26 Göteborg (SE)
(72) Inventor: BLOCK, Michael, Metairie, LA 70002 (US); HOFFMAN, David R., Metairie, LA 70002 (US)
(74) Representative: Popp, Eugen, Dr.
(86) International application number: PCT/US92/01431
(87) International publication number: WO 92/14417

(56) References cited:
- FR-A- 2 631 813
- US-A- 3 082 525
- US-A- 4 379 694
- US-A- 4 702 697
- US-A- 5 052 930
- US-A- 5 085 660
- US-A- 5 087 259

## Description

Skeletal deformities become evident during the growth of an individual, or may be acquired from trauma, tumor resection, or systemic disease.

Correction of bone deformities requires either surgical treatment to reposition the deformed bones into a "normal" relationship, or by guided bone movements. Currently pins or other transosseous devices are used in conjunction with surgical procedures to anchor the bones and to maintain bone position during treatment and healing. These transosseous devices have limitations, such as in small bones and in regions of the human skeleton such as the face where other vital structures exist preventing pins from being used. The correction of facial deformities presents clinical challenges which have led to this invention.

In patients with atrophic maxillary or mandibular bone, prosthetic rehabilitation with conventional dentures is often not satisfactory since the patient has very little bone to retain the dentures. In order to rehabilitate these patients, bone grafting is often required. However, many patients are not candidates for bone grafting due to health reasons. Their rehabilitation requires only an anchor for improved retention of their prosthesis.

Often the earliest signs of maxillary or mandibular growth disharmony is dental malalignment. Once recognized, it is possible to guide the growth of segments of the cranio-facial skeleton in order to minimize the need for surgical correction of the deformity.

Maxillary hypoplasia exists in all three dimensions. Transverse deficiency of the maxilla is often treated by the orthodontist with orthopedic palatal expansion. Deficiency in the maxillary in the vertical or anterior-posterior direction has not been satisfactorily cured by non-surgical guided movements because of a lack of a stable or non-mobile anchorage source for orthopedic movements.

Mandibular deficiency can be corrected by functional appliances which position the mandible forward, and presumably allow for posterior condylar appositional growth which stabilizes the mandible in this forward position. Orthodontists employ orthopedic traction in all three dimensions to control or direct the development of a bone to a favorable location.

Cleft palate patients often have transverse, anterior-posterior, and vertical dysplasia. Reconstruction of these patients often involves orthodontic alignment of the segments prior to bone grafting the defects. However, the defects can be large and difficult to manage when the patient is young. The deciduous dentition can also be difficult to manage in regards to orthodontic anchorage preventing definitive alignment of the arches until the patient is in the early teens.

All orthodontic and orthopedic forces adhere to Newton's Law of Reciprocal Forces. If a force is applied to retract, or pull back an object such as a tooth, there exists an "equal and opposite" force to move another tooth forward. The resistive value of the posterior teeth is known as anchorage.

Orthodontists offset these reciprocal tendencies by using an extraoral force known as a headgear to augment the resistive value of the molar teeth. However, patient compliance may be poor because many patients do not want to wear the headgear, compromising orthodontic therapy and often the final result.

The problem is that the retractive forces are usually continuous, acting 24 hours a day. Realistically most patients will not wear a headgear more than 10 - 12 hours a day. Therefore, the posterior anchorage is typically fortified 40 to 50% of the time. All too often inconsistent usage or overt non-compliance reduce this effect even more.

Previous work in this field indicates that endosseous implants can be used to anchor orthodontic forces for tooth movement. All of the previously used implants were cylindrical or screw shaped, from eight to 22 mm in length. These studies indicate that osseointegrated implants have been used to anchor realignment of teeth, without moving the implants. These implants were placed deeply into the bone.

In the field of orthopedics, pins are routinely placed through bones and connected to various supporting frameworks to maintain bone position and also to act as an anchor for guided bone movements. Morbidity is associated with placing pins through the cortical and cancellous bone, and if complications such as pin loosening or infection occurs, loss of bone structure can occur.

Clinically, hydroxylapatite coated cylindrical implants have been used since July 1984. Solid blocks of dense hydroxylapatite are available for interpositional and onlay grafting of defects during orthognathic surgery. The onlay grafts were used exclusively for cosmetic augmentation of facial defects without carrying loads.

The French patent publication FR-2,631,813 discloses a dental implant comprising a sheet-like base to be fitted over the jaw bone. The plate is covered with bioactive material for providing biointegration with the bone. A central portion of the implant comprises a bore designed to receive a dental prosthesis.

The object of the present invention is to provide an improved anchor system including a bone anchor and an orthodontic device for treating malalignment of the teeth. The bone anchor or onplant should be thin enough to allow bone overgrowth and should provide sufficient sheer strength to absorb chewing forces and other forces from orthodontic loading.

According to the present invention, a bone anchor system is provided as defined in Claim 1. The system comprises a bone anchor or onplant which is surgically placed on a bone or in a shallow depression thereby allowing biointegration. The depression will preferably be about 3 mm to provide sufficient anchorage. Placement of the bioactively coated bone interface will allow bone growth over the entire surface of the 2-3 mm thin device. The bone growth increases the ability of the bone anchor to withstand the forces of chewing.

The orthodontic device may be a palatal bar which is attached to the bone anchor or onplant, the palatal bar having two bands to be placed around two teeth. The two teeth are held non-mobile, thereby permitting the orthodontist to treat the malalignment of the remaining teeth.

As a prosthetic anchor the thin, completely bioactively coated bone anchor or onplant is surgically placed in a shallow depression in the bone to allow for bone to heal on all of the onplant's surfaces, allowing biointegration between the onplant bone interface and the bone, after which an attachment device is attached to the onplant to provide anchorage for orthodontic devices. The optional placement of the completely bioactively coated onplant into a shallow depression on the bone is for extended applications.

The onplant may have a screw hole penetrating it for the sole purpose of stabilizing it with a small screw into the bone while the onplant is integrating. The screw would serve no purpose once integration had occurred and may be removed when the surgeon exposes the onplant to attach the intended device.

The invention will be better understood and the objects other than those set forth above will become apparent when consideration is given to the following detailed description of embodiments. Such description makes reference to the annexed drawings wherein:
FIGURE 1 is a perspective view of the orthodontic anchor of one embodiment;
FIGURE 2 is an exploded side elevation view of the embodiment of FIGURE 1;
FIGURE 3 is a top view of the embodiment of FIGURE 1;
FIGURE 4 is a bottom view of FIGURE 1;
FIGURE 5 is a bottom view of an alternative embodiment of the orthodontic anchor;
FIGURE 6 is a bottom view of an alternative embodiment of the orthodontic anchor;
FIGURE 7 is a bottom view of an embodiment with the orthodontic anchor system installed in the roof of a mouth with the palatal wire connected to two banded teeth;
FIGURE 8 is a bottom view with another embodiment installed in the roof of a mouth with the palatal bar connected to two banded teeth.
FIGURE 9 is a human skull with bone anchors which is not part of the invention;
FIGURE 10 is the mandible or lower jaw shown in FIGURE 9;
FIGURE 11 is a cross-sectional view taken on lines II-II of FIGURE 10;
FIGURE 12 is a partial cross-sectional view of the upper jaw of a living person;
FIGURE 13 is a cross-sectional of a tibia or leg bone showing a bone anchor;
FIGURE 14 is a finger bone with a bone anchor; and,
FIGURE 15 is a rib bone with a bone anchor.

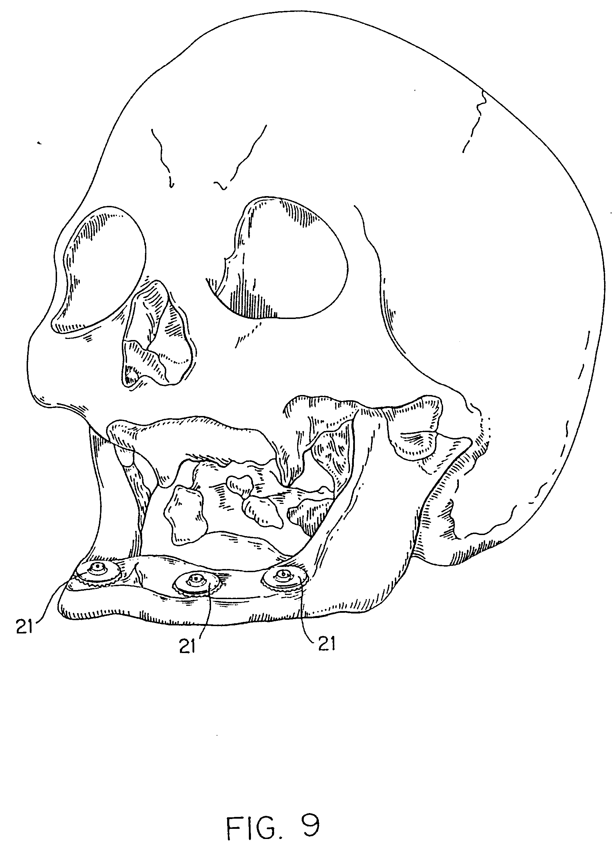

Figures 9 to 15 illustrate other uses of bone anchors, while these illustrations are not embodiments of the claimed invention.

### DESCRIPTION OF THE PREFERRED EMBODIMENTS

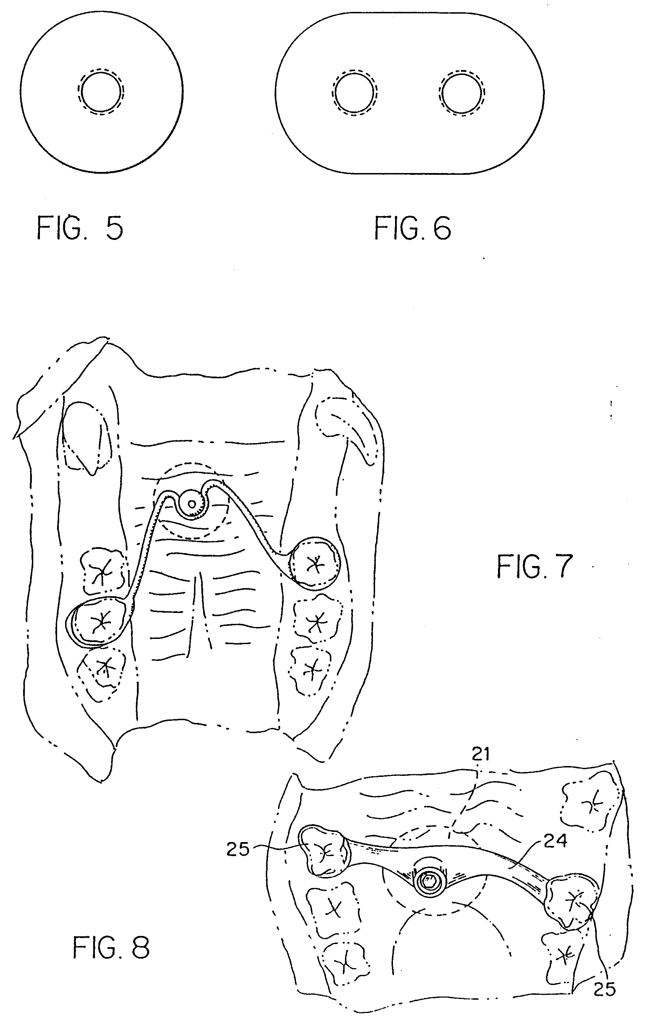

The orthodontic anchor 20 has two parts; the onplant or bone anchor 21 and the abutment 22. These are connected to a palatal bar 24 or palatal wire 28, which is attached to bands 25 around the teeth to be held immobile (Figs. 7, 8).

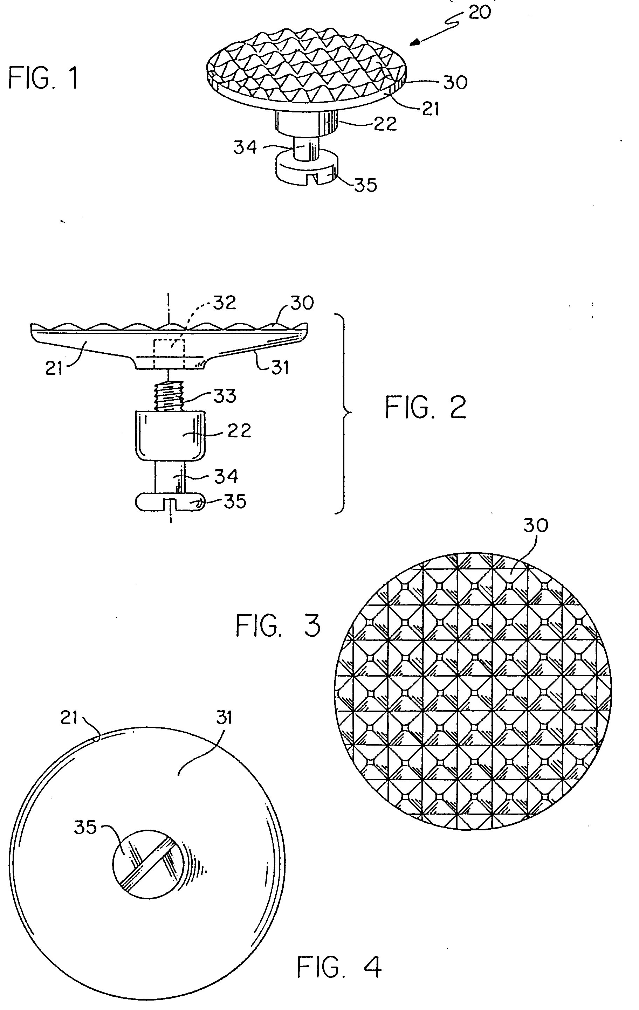

As show in FIGURES 1-4 the onplant 21 has an circular upper surface which is the onplant bone interface 30. The circular shape is illustrative only. The onplant may be an oval, a square, a rectangle, a triangle, or other shape to resist the forces applied to it. This onplant bone interface 30 is textured, which both increases the surface area and presents surface area which is better able to resist the shear forces imposed by the orthodontic anchor system 20. Both the textured onplant bone interface 30 and the surface 31 is covered with hydroxylapatite or other bioactive material.

The onplant 21 has a lower surface with a beveled outer portion 31 and a central circular portion 36. The outer portion 31 joins the outer periphery of the onplant bone interface 30. The center of the lower surface 36 has at least one threaded aperture 32. There may be more than one threaded aperture depending on the need to resist rotational forces. When the onplant 21 is initially installed the threaded aperture 32 has a healing screw 26, not shown, installed to prevent tissue from covering it and having to be removed.

The abutment 22 is circular, with an upper surface 37 matching the lower surface 36 of the onplant 21. The upper surface 37 has a protruding threaded screw 33 which cooperates with the threaded aperture 32. The abutment 22 has a neck 34 of reduced diameter and a head 35 of increased diameter, compared with the neck 34. Surface 35 as shown is illustrative only. It may have a slot, hexagonal, or threaded hole or other means of seating the abutment or attaching the palatal bar.

The dimensions of the onplant 21 may be 8 mm in diameter and 2 mm thickness. The abutment 22 may be 4 mm in overall height, with the neck 34 being 1 mm in height and 1 mm in diameter. The device will vary in size according to the shape and the designed force load.

The material of both the onplant 21 and the abutment 22 is a titanium alloy. The surface, except for the onplant bone interface 30, is smooth and all corners are beveled to prevent damage to soft tissue.

The test sample had a 50 micron coating of hydroxylapatite. It was plasma sprayed on the metal. The spray consists of a superheated solution of hydroxylapatite applied to the roughened titanium alloy.

FIGURE 5 shows a onplant 21 which is similar to the onplant 21 of FIGURE 1. This onplant 21 is generally cylindrical in shape. It has the onplant bone interface 30 which is textured and coated by hydroxylapatite, and 3 threaded aperture 32.

FIGURE 6 is similar to FIGURE 5, but is oval in shape and has two threaded apertures 32. This embodiment permits two orthodontic devices to be used.

As shown in FIGURE 7 the palatal wire 28 is soldered to the two bands 25 of two molars or other teeth and presses into the neck 34 of the abutment 22, preventing the two teeth from moving forward. This palatal wire 28 may be fabricated from 1.3 mm (0.051 in.) orthodontic wire or cast from precious or non-precious metals.

FIGURE 8 shows the orthodontic anchor system 20 mounted between the two teeth to be held stable. The palatal bar 24 is fabricated from thicker metal to resist the shear forces of the teeth against the orthodontic anchor system 20. This palatal bar 24 is screwed on abutment 22 which is screwed into the onplant 21.

The orthodontic anchor system 20 is able to resist both primary lateral and horizontal forces as well as a vertical force.

The orthodontic anchor system 20 is not limited to use with a palatal bar 24. It may alternatively be used with any conventional orthodontic device, as will be immediately apparent.

It is within the scope of the invention to use other suitable materials for the orthodontic anchor system 20. These will include inert metals, plastics and composites. Likewise the bonding means can be any mechanical means such as keylocks or miters or magnetic or biodegradable polymer. The onplant bone interface 30 may have a different textured surface or a non-textured surface which promotes adequate bonding strength. The thickness and method of applying the hydroxylapatite coating may be varied.

The orthodontic anchor system 20 is installed into a patient's mouth in accordance with the following procedures. These are generalized for an understanding of the invention, and are not the detailed procedures which would be actually followed by a surgeon.

Under local anaesthesia, an anterior palatal incision will be made and a subperiosteal tunnel created so that the tunnel will place the onplant at the proposed location (most likely between the permanent first molars). Conservative dissection will be used in order that palatal reflection is minimal and restricted to only the onplant site in order to prevent onplant migration. One or two onplants will be placed depending on the treatment needs for the patient.

For prosthetic or extended applications, an incision will be made along the crest of the alveolar bone, and the periosteum will be reflected. A precision bur may be used to create an optional shallow depression identical to the shape of the anchor, to the depth required so the anchor lies flush with the bone.

Previous experience indicates that careful surgical technique will result in secure positioning of these onplants, without the need for retentive wires to maintain bone contact on flat surfaces. However, on curve surfaces a small screw or suture may be needed to retain the onplant in the preferred position during biointegration.

The onplant is usually provided sterile by the manufacturer. It will be placed into the subperiosteal tunnel taking great care to place it directly against the palatal or other bone, or into the shallow depression within the bone. The incision will be closed using 4-0 polyglactin suture. The patients will be given a prescription for antibiotics (typically penicillin or doxyclycline) and analgesics. This small surgical procedure should cause minimal pain to the patient. The patient will be called at home by the surgeon for follow up, and seen for suture removal one week after the surgery. The patient will be followed every two weeks for observation during the healing period which is necessary to achieve integration of the hydroxylapatite surface with the underlying bone.

Twelve weeks will be allowed for healing and biointegration to occur. Twelve weeks is the expected onplant biointegration time because that is the time required for integration of hydroxylapatite coated implants in humans. At twelve weeks, the patients will be given local anaesthesia and a small incision will be made directly over the onplant, exposing only the healing screw 26 that was placed into the internal thread of each device. An abutment 22 is then screwed into the onplant. The overlying soft tissue thickness may be thinned to 3 mm in order to allow for cleaning of the attachment device. An impression will be taken in order to fabricate a palatal bar 24 which is secured to the onplant and banded to the dentition.

The palatal wire 28 will be solid and minimally pliable. The wire will be soldered to bands glued to the anchor teeth. Approximately two weeks will be allowed for fabrication of the bar or bending the wire on a transferred study model. The wire will be fabricated of 1.3 mm (0.051 in.) orthodontic wire.

Two weeks later, orthodontic devices will be attached to the onplant and to the maxillary teeth, for example the first molar, placed in such a way that the wire attaching the onplant to the tooth acts to hold the tooth in position, as an anchor. The onplant will serve as the point of absolute anchorage, preventing the anchored teeth from moving anteriorly.

The remaining dentition will be treated with conventional orthodontic appliances. The location of the teeth with respect to the onplants may be measured and recorded both by radiographs and actual physical measurement with a Boley gauge.

At the conclusion of the treatment involving the device, under local anesthesia, an incision will be made exposing the entire onplant. Using a forcep designed for this procedure, the hydroxylapatite coated device will be removed. The prosthetic device will be left in place to provide anchorage of the denture.

In a study investigating the difference between diameter and length on the ultimate pull-out strength of hydroxylapatite coated cylinders in the dog jaw, a mechanically significant bonding was found with hydroxylapatite coated implants. In the dog alveolus in a cortical and cancellous bone environment, up to 45 pounds were required to pull hydroxylapatite coated onplants from the dog jaw. Based on these mechanical studies of onplants, we are confident that the onplant's hydroxylapatite bone bond can withstand continuously applied forces.

Hydroxylapatite coated implants can be used for restoration of partially and totally edentulous patients. Occlusal function has not resulted in loss of the hydroxylapatite coating, thus continuous occlusal function helps confirm our belief that a hydroxylapatite coated device can function under continuous load.

To further verify this concept, clinical trial of using hydroxylapatite coated dental implants as orthodontic anchor systems 20 (Hoffman, Block personal communication, 1989) demonstrate that one or two hydroxylapatite coated implants placed within the bone of the maxilla or mandible can be used as anchors for tooth movement. Teeth attached to the implants did not move whereas those teeth not attached to the implants moved noticeably when subjected to a similar force. Both in animal studies and in these clinical trials, these implants did not move, rather the teeth were moved when constant forces in excess of 11 ounces were continuously placed on the implants.

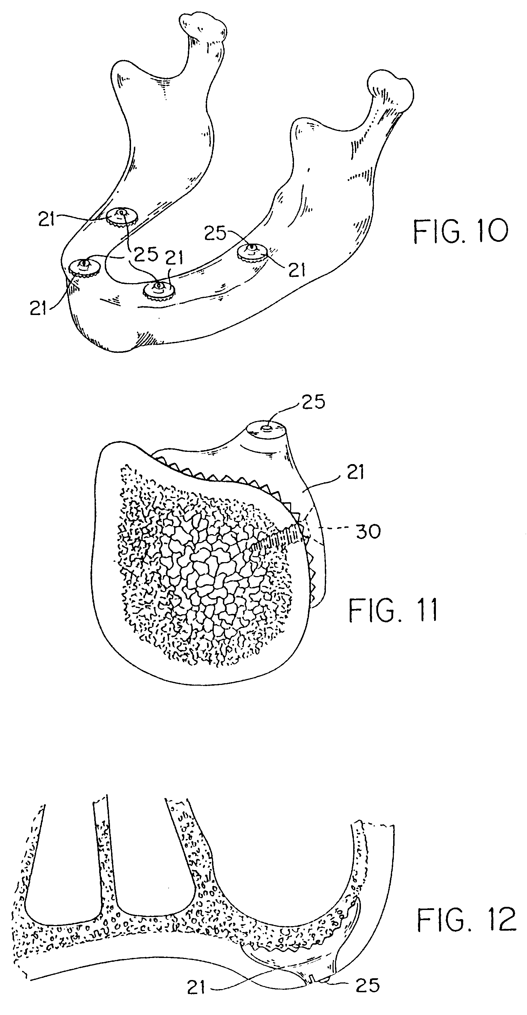

Figures 9-15 show other uses of bone anchors. FIGURE 9 shows a skull of an older male who has lost his teeth, and whose jawbone has partially atrophied. FIGURE 10 shows the mandible of the skull with four onplants 21 attached. FIGURE 11 shows the onplant shaped to the surface contour of the mandible bone. It discloses a integration stabilization screw 30 which may be used while biointegration occurs. On a curved surface drifting or lack of intimate contact may require this small retaining screw during the biointegration process. The screw serves no other purpose than to stabilize the onplant during this process and may be removed after biointegration.

FIGURE 12 shows the upper jawbone of a living person between the gingiva or gum and the maxillary sinus. The loss of teeth atrophies the bone, often making the known implants impossible to use because of the thinness of the upper jawbone. An onplant 21 is shown biointegrated to the upper jawbone.

The surface of the onplant which will rest against the bone may be textured, as shown, to increase the surface and resist shear forces. This is not necessary as a smooth surface which biointegrates with sufficient strength may also be used. That surface of the onplant, if an inactive metal, must be covered with a bioactive material, such as hydroxylapatite, to promote biointegration.

For the prosthetic application, an incision is made along the crest of the alveolar bone and the periosteum is reflected. The surface of the onplant which will rest against the bone is shaped to the contour of the bone. The onplant may be held firmly against the bone by the use of small resorbable screws. A shallow depression may be cut in the bone to allow the onplant to lie flush with the surface of the bone.

The surface of the onplant opposite the surface facing the bone will have means 25 to attach any of the current conventional dental protheses, including dentures. This means may be, for instance, a ball, a ring, or a magnet, which will cooperate with the mounting on the prosthesis to stabilize the prosthesis.

The bone anchor may be used with an orthopedic device in order to guide the movement of bones to bring them either closer together or further apart, for the correction of bone deformities or injuries. This application is not part of the present invention.

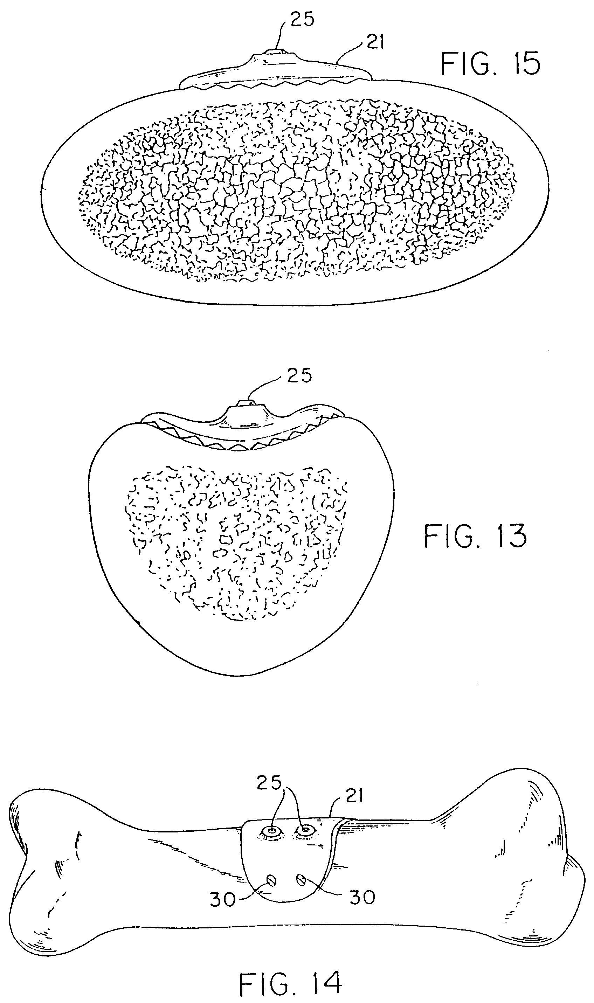

Onplants 21 are placed in subperiosteal tunnels in two or more bones. The onplant 21 may have to be shaped to either a convex shape to fit a tibia bone, see FIGURE 13, or a concave shape to fit a finger bone, see FIGURE 14. In certain cases, such as the finger bone, the onplant could be held in place during biointegration by sutures or the aformational integration stabilization screws.

After biointegration, orthopedic devices are attached to two or more onplants on different bones in tension or compression, to transmit attractive or distractive forces for bone reconstruction. Alternatively the onplant 21, such as that on a finger bone, as in FIGURE 14, could be used as a prosthetic anchor for an artificial finger tip.

A bone anchor could be used to attach a medical device. FIGURE 15 shows an onplant 21 on a rib bone. A pacemaker may be attached to it, which would provide more stable mounting than is conventionally used today.

## Claims

1. A prosthetic bone anchor system, comprising:
a bone anchor (21) disposable on the surface of the bone or into a shallow depression in the bone;
said anchor (21) having a first surface (30) shaped to match the surface of the bone, said first surface (30) being covered with a bioactive material for providing bio-integration with the bone, and
attachment means (22; 32 - 35) for attaching a prosthetic device on a second surface (31) of the anchor (21) opposite said first surface (30);
characterized in that
said prosthetic device is an orthodontic device (24, 28) attached to two bands (25) placeable around two teeth,
said attachment means (22; 32 - 35) include abutment means (22) for securing said orthodontic device (24, 28) to said bone anchor (21).

2. The system of Claim 1, wherein said orthodontic device is a palatal wire (28).

3. The system of Claim 1, wherein said orthodontic device is a palatal bar (24).

## Patentansprüche

1. Prothetisches Knochenverankerungssystem umfassend:
eine Verankerung (21), die an der Oberfläche des Knochens oder in einer seichten Vertiefung des Knochens anbringbar ist;
wobei die Verankerung (21) eine erste Oberfläche (30) besitzt, die so geformt ist, daß sie mit der Oberfläche des Knochens zusammenpaßt, und die erste Oberfläche (30) zur Biointegration mit dem Knochen mit einem bioaktiven Material beschichtet ist, und
Befestigungselemente (22; 32-35) zum Befestigen einer prothetischen Vorrichtung auf einer zweiten Oberfläche (31) der Verankerung (21), die der ersten Oberfläche (30) gegenüberliegt;
dadurch gekennzeichnet, daß die prothetische Vorrichtung eine kieferorthopädische Vorrichtung (24,28) ist, die an zwei um zwei Zähne anbringbare Bänder (25) befestigt ist, wobei das Befestigungselement (22; 32-35) Befestigungsmittel (22) zur Befestigung der kieferorthopädischen Vorrichtung (24, 28) an die Knochenverankerung (21) aufweist.

2. System nach Anspruch 1, dadurch gekennzeichnet, daß die kieferorthopädische Vorrichtung ein Gaumendraht (28) ist.

3. System nach Anspruch 1, dadurch gekennzeichnet, daß die kieferorthopädische Vorrichtung ein Gaumenband (24) ist.

## Revendications

1. Système d'ancre à os prothétique, comprenant :
une ancre à os (21) pouvant être placée sur la surface de l'os ou dans une cavité peu profonde faite dans l'os,
cette ancre (21) ayant une première surface (30) façonnée pour épouser la surface de l'os, cette première surface (30) étant recouverte d'une matière bioactive pour produire une biointégration à l'os, et
un moyen de fixation (22 ; 32 à 35) pour la fixation d'un dispositif prothétique sur une deuxième surface (31) de l'ancre (21) opposée à la première surface (30),
caractérisé par le fait que
le dispositif prothétique est un dispositif orthodontique (24, 28) fixé à deux bagues (25) pouvant être placées autour de deux dents,
le moyen de fixation (22 ; 32 à 35) comprend un moyen de butée (22) pour la fixation du dispositif orthodontique (24, 28) à l'ancre à os (21).

2. Système selon la revendication 1, dans lequel le dispositif orthodontique est un fil palatin (28).

3. Système selon la revendication 1, dans lequel le dispositif orthodontique est une barre palatine (24).
